# EUROPEAN PATENT SPECIFICATION

(11) **EP 3 919 899 B1**
(45) Date of publication and mention of the grant of the patent: **10.09.2025**
(21) Application number: 19933546.4
(22) Date of filing: 18.12.2019
(51) Int. Cl.: G01N 33/00

(54) **FORMALDEHYDE CONCENTRATION MEASUREMENT METHOD AND APPARATUS, AND AIR PURIFIER**
VERFAHREN UND VORRICHTUNG ZUR MESSUNG DER FORMALDEHYDKONZENTRATION UND LUFTREINIGER
PROCÉDÉ ET APPAREIL DE MESURE DE CONCENTRATION DE FORMALDÉHYDE, ET PURIFICATEUR D'AIR

(30) Priority: 21.06.2019 CN 201910544470
(43) Date of publication of application: 08.12.2021
(73) Proprietor: Gree Electric Appliances, Inc. of Zhuhai, Zhuhai, Guangdong 519070 (CN)
(72) Inventor: KANG, Zhengwei, Zhuhai, Guangdong 519070 (CN); NI, Linhai, Zhuhai, Guangdong 519070 (CN); QIN, Bin, Zhuhai, Guangdong 519070 (CN); SU, Weibang, Zhuhai, Guangdong 519070 (CN)
(74) Representative: Appleyard Lees IP LLP
(86) International application number: PCT/CN2019/126167
(87) International publication number: WO 2020/253175

(56) References cited:
- EP-B1- 3 111 229
- CN-A- 103 646 591
- CN-A- 103 646 591
- CN-A- 107 884 364
- CN-A- 109 655 518
- CN-A- 109 655 518
- CN-A- 109 655 518
- CN-A- 109 669 008
- CN-A- 110 320 328
- CN-U- 203 299 122
- CN-U- 208 170 581
- KR-A- 20140 091 847
- US-A- 6 128 945
- US-A1- 2013 189 791
- US-B2- 9 857 243

## Description

### FIELD

The present invention relates to the technical field of measurement equipment, and in particular to a formaldehyde concentration measurement method and apparatus, and an air purifier.

### BACKGROUND

Air quality measurement equipment is usually equipped with an air quality sensor which is used to measure air quality in the surrounding environment. For example, a formaldehyde sensor is used to measure the content of formaldehyde gas in the air to obtain a formaldehyde concentration value. With the operation of the formaldehyde sensor, due to the changes of factors such as temperature and humidity in the surrounding environment, the formaldehyde concentration values measured by the formaldehyde sensor in the environments with different temperatures and humidity levels are different, resulting in errors of the formaldehyde concentration value measured in different environments. CN109655518A discloses a calibration method for formaldehyde electrochemical sensing detection apparatus. CN103646591A discloses a sensor zero point learning method. The sensor is applied to an electric power steering (EPS) system. US9857243B2 discloses a self-correcting chemical sensor.

### SUMMARY

In view of the above, the present invention provides a formaldehyde concentration measurement method and apparatus, and an air purifier, which are capable of reducing errors of formaldehyde concentration value measured by a formaldehyde sensor in different environments, thereby improving the measurement accuracy of the formaldehyde sensor.

In view of this, in a first aspect, the present invention provides a formaldehyde concentration measurement method, including steps of:
determining an initial formaldehyde concentration value measured by a formaldehyde sensor;
acquiring temperature information and/or humidity information of an environment surrounding the formaldehyde sensor;
determining a formaldehyde concentration compensation value of the formaldehyde sensor according to the temperature information and/or the humidity information; and
determining a target formaldehyde concentration value of the formaldehyde sensor according to the formaldehyde concentration compensation value and the initial formaldehyde concentration value.

With reference to the first aspect, in some embodiments, the temperature information is a temperature value, the humidity information is a humidity value, and the step of determining the formaldehyde concentration compensation value of the formaldehyde sensor according to the temperature information and/or the humidity information includes steps of:
calculating a first difference between the temperature value and a set temperature threshold, and/or calculating a second difference between the humidity value and a set humidity threshold; and
determining the formaldehyde concentration compensation value of the formaldehyde sensor according to the first difference and/or the second difference.

With reference to the first aspect, in some embodiments, the step of determining the formaldehyde concentration compensation value of the formaldehyde sensor according to the first difference and/or the second difference includes steps of:
determining a first formaldehyde concentration compensation value of the formaldehyde sensor corresponding to the first difference between the temperature value and the set temperature threshold, according to a preset corresponding relationship between the first difference, between the temperature value and the set temperature threshold, and the formaldehyde concentration compensation value of the formaldehyde sensor;
   and/or
determining a second formaldehyde concentration compensation value of the formaldehyde sensor corresponding to the second difference between the humidity value and the set humidity threshold, according to a preset corresponding relationship between the second difference, between the humidity value and the set humidity threshold, and the formaldehyde concentration compensation value of the formaldehyde sensor; and
setting the first formaldehyde concentration compensation value, or the second formaldehyde concentration compensation value, or a sum of the first formaldehyde concentration compensation value and the second formaldehyde concentration compensation value, as the formaldehyde concentration compensation value of the formaldehyde sensor.

With reference to the first aspect, the method further includes steps of:
acquiring operating parameter information of the formaldehyde sensor in a preset time period, wherein the operating parameter information includes: total operating time period of the formaldehyde sensor in the preset time period, and/or total number of operations of the formaldehyde sensor in the preset time period; and
sending a corresponding command signal to the formaldehyde sensor in order to instruct the formaldehyde sensor to perform zero point calibration when the operating parameter information satisfies a preset condition.

With reference to the first aspect, in some embodiments, the step of sending the corresponding command signal to the formaldehyde sensor in order to instruct the formaldehyde sensor to perform the zero point calibration, when the operating parameter information satisfies the preset condition, includes steps of:
comparing the total operating time period of the formaldehyde sensor in the preset time period with a set total operating time period threshold to obtain a first comparison result; and/or
comparing the total number of operations of the formaldehyde sensor in the preset time period with a set total number threshold of operations to obtain a second comparison result;
determining that the operating parameter information satisfies the preset condition when the first comparison result is: the total operating time period of the formaldehyde sensor in the preset time period is greater than the set total operating time period threshold, and/or the second comparison result is: the total number of operations of the formaldehyde sensor in the preset time period is greater than the set total number threshold of operations.

In a second aspect, the present invention provides a formaldehyde concentration measurement apparatus, including a processor, a communication interface, a formaldehyde sensor, and a temperature sensor and/or a humidity sensor; the formaldehyde sensor and the temperature sensor and/or the humidity sensor are electrically connected to the processor via the communication interface.

The formaldehyde sensor is configured to measure a concentration of formaldehyde to obtain an initial formaldehyde concentration value, and send the initial formaldehyde concentration value to the processor.

The temperature sensor is configured to collect temperature information of an environment surrounding the formaldehyde sensor, and send the temperature information to the processor.

The humidity sensor is configured to collect humidity information of the environment surrounding the formaldehyde sensor, and send the humidity information to the processor.

The processor is configured to determine a formaldehyde concentration compensation value of the formaldehyde sensor according to the received temperature information and/or humidity information, and to determine a target formaldehyde concentration value of the formaldehyde sensor according to the formaldehyde concentration compensation value and the received initial formaldehyde concentration value.

With reference to the second aspect, in some embodiments, the temperature information is a temperature value, the humidity information is a humidity value, and the processor includes:
a difference calculation module configured to calculate a first difference between the temperature value and a set temperature threshold, and/or calculating a second difference between the humidity value and a set humidity threshold; and
a compensation value determination module configured to determine the formaldehyde concentration compensation value of the formaldehyde sensor according to the first difference and/or the second difference calculated by the difference calculation module.

With reference to the second aspect, in some embodiments, the compensation value determination module includes:
a first compensation value determination unit configured to determine a first formaldehyde concentration compensation value of the formaldehyde sensor corresponding to the first difference between the temperature value and the set temperature threshold, according to a preset corresponding relationship between the first difference, between the temperature value and the set temperature threshold, and the formaldehyde concentration compensation value of the formaldehyde sensor; and/or
a second compensation value determination unit configured to determine a second formaldehyde concentration compensation value of the formaldehyde sensor corresponding to the second difference between the humidity value and the set humidity threshold, according to a preset corresponding relationship between the second difference, between the humidity value and the set humidity threshold, and the formaldehyde concentration compensation value of the formaldehyde sensor; and
a formaldehyde concentration compensation value determination unit configured to set the first formaldehyde concentration compensation value determined by the first compensation value determination unit, or the second formaldehyde concentration compensation value determined by the second compensation value determination unit, or a sum of the first formaldehyde concentration compensation value determined by the first compensation value determination unit and the second formaldehyde concentration compensation value determined by the second compensation value determination unit, as the formaldehyde concentration compensation value of the formaldehyde sensor.

With reference to the second aspect, the processor further includes:
a parameter information acquisition module configured to acquire operating parameter information of the formaldehyde sensor in a preset time period. The operating parameter information includes: total operating time period of the formaldehyde sensor in the preset time period, and/or total number of operations of the formaldehyde sensor in the preset time period.

The processor sends a corresponding command signal to the formaldehyde sensor in order to instruct the formaldehyde sensor to perform zero point calibration when the operating parameter information acquired by the parameter information acquisition module satisfies a preset condition.

With reference to the second aspect, in some embodiments, the processor further includes:
a first comparison module configured to compare the total operating time period of the formaldehyde sensor in the preset time period with a set total operating time period threshold to obtain a first comparison result; and/or
a second comparison module configured to compare the total number of operations of the formaldehyde sensor in the preset time period with a set total number threshold of operations to obtain a second comparison result.

The processor determines that the operating parameter information acquired by the parameter information acquisition module satisfies the preset condition, on a condition that the first comparison result obtained by the first comparison module is: the total operating time period of the formaldehyde sensor in the preset time period is greater than the set total operating time period threshold, and/or on a condition that the second comparison result obtained by the second comparison module is: the total number of operations of the formaldehyde sensor in the preset time period is greater than the set total number threshold of operations.

In a third aspect, the present invention provides an air purifier, including the formaldehyde concentration measurement apparatus as described in the second aspect.

The embodiments of the present invention provide a formaldehyde concentration measurement method and apparatus, and an air purifier. The formaldehyde concentration measurement method includes steps of: determining an initial formaldehyde concentration value measured by a formaldehyde sensor; acquiring temperature information and/or humidity information of an environment surrounding the formaldehyde sensor; determining a formaldehyde concentration compensation value of the formaldehyde sensor according to the temperature information and/or the humidity information; and determining a target formaldehyde concentration value of the formaldehyde sensor according to the formaldehyde concentration compensation value and the initial formaldehyde concentration value.

According to the formaldehyde concentration measurement method, errors of the formaldehyde concentration value measured by the formaldehyde sensor in different environments can be reduced, thereby improving the measurement accuracy of the formaldehyde sensor. For example, the formaldehyde concentration compensation value of the formaldehyde sensor is calculated according to the temperature information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different temperature environments. In another example, the formaldehyde concentration compensation value of the formaldehyde sensor is calculated according to the humidity information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different humidity environments. In yet another example, the formaldehyde concentration compensation value of the formaldehyde sensor is calculated according to the temperature and humidity information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different temperature and humidity environments.

### BRIEF DESCRIPTION OF THE DRAWINGS

In order to more clearly illustrate the technical solutions in the embodiments of the present invention or in the prior art, the drawings that are to be referred in the description of the embodiments or the prior art are briefly described below. Obviously, the drawings in the following description are only show embodiments of the present invention. For those of ordinary skill in the art, other drawings can be obtained according to the disclosed drawings without any creative efforts.
FIG. 1 is a schematic flow chart of a formaldehyde concentration measurement method provided in some embodiments of the present invention.
FIG. 2 is a schematic flow chart of another formaldehyde concentration measurement method provided in some embodiments of the present invention.
FIG. 3 is a schematic flow chart of yet another formaldehyde concentration measurement method provided in some embodiments of the present invention.
FIG. 4 is a schematic structural view of a formaldehyde concentration measurement apparatus provided in some embodiments of the present invention.

### DETAILED DESCRIPTION

In order to make the objective, the technical solutions, and the merits of the embodiments of the present invention clear, the technical solutions in the embodiments of the present invention are clearly and completely described below in conjunction with the drawings in the embodiments of the present invention. Obviously, the embodiments described are only some, rather than all, of the embodiments of the present invention. All other embodiments obtained by a person of ordinary skill in the art based on the embodiments in the present invention without creative efforts are within the scope of the present invention.

A server implementing various embodiments of the present invention will now be described with reference to the accompanying drawings. Therefore, "module" and "component" may be used interchangeably.

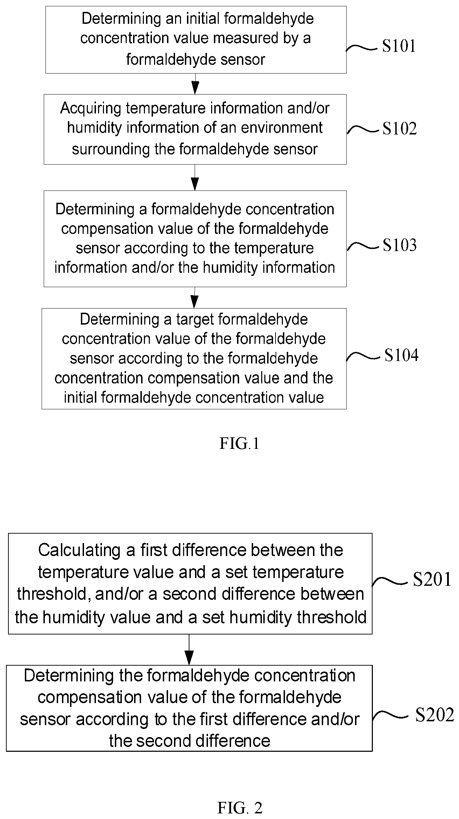

As shown in FIG. 1, some embodiments of the present invention provide a formaldehyde concentration measurement method, including the following steps.

At step S101, an initial formaldehyde concentration value measured by a formaldehyde sensor is determined.

The embodiments of the present invention are applied to the technical field of measurement equipment. Specifically, when the formaldehyde sensor in the air purifier is used to measure the formaldehyde content in the air, the formaldehyde concentration value measured in environments with different temperatures and humidity levels can be corrected according to the temperature information and the humidity information of the environment surrounding the formaldehyde sensor, so as to reduce measurement errors of the formaldehyde sensor in different environments. The embodiments of the present invention can also be applied to the field that air quality is measured by using other air quality sensors in the air quality detection equipment, which is not limited herein.

The initial formaldehyde concentration value in some embodiments of the present invention is an initial measurement value of the formaldehyde concentration obtained by measuring the formaldehyde content in the air by the formaldehyde sensor in the current temperature and humidity environment, i.e., an initial measurement value that has not been corrected according to the temperature and humidity information of the surrounding environment.

At step S102, temperature information and/or humidity information of the environment surrounding the formaldehyde sensor is acquired.

In some embodiments of the present invention, the temperature information is a temperature value, and the humidity information is a humidity value. Both the temperature value and the humidity value are environmental factors that affect the initial formaldehyde concentration value measured by the formaldehyde sensor. In an embodiment, only one environmental factor, e.g., the temperature value, may be considered, and the formaldehyde concentration compensation value of the formaldehyde sensor is calculated according to the temperature value. In another embodiment, only another environmental factor, e.g., the humidity value, may be considered, and the formaldehyde concentration compensation value of the formaldehyde sensor is calculated according to the humidity value. In yet another embodiment, the two environmental factors, i.e., the temperature value and the humidity value, may be considered simultaneously, and the formaldehyde concentration compensation value of the formaldehyde sensor is calculated according to the temperature value and the humidity value.

In some embodiments of the present invention, the temperature value of the environment surrounding the formaldehyde sensor is measured by a temperature sensor, and the humidity value of the environment surrounding the formaldehyde sensor is measured by a humidity sensor.

At step S103, a formaldehyde concentration compensation value of the formaldehyde sensor is determined according to the temperature information and/or the humidity information.

The formaldehyde concentration compensation value calculated in some embodiments of the present invention is used to correct the initial formaldehyde concentration value measured by the formaldehyde sensor. The formaldehyde concentration compensation value may be a positive value or a negative value. The specific calculation of the formaldehyde concentration compensation value is illustrated in the following steps.

At step S104, a target formaldehyde concentration value of the formaldehyde sensor is determined according to the formaldehyde concentration compensation value and the initial formaldehyde concentration value.

In some embodiments of the present invention, corresponding formaldehyde concentration compensation values are calculated according to the temperature values of the surrounding environment in different temperatures and the humidity values of the surrounding environment in different humidity levels, and the initial formaldehyde concentration value with large error and low accuracy is corrected by using the formaldehyde concentration compensation value, so as to obtain the target formaldehyde concentration value with smaller error and higher accuracy.

According to the formaldehyde concentration measurement method provided in some embodiments of the present invention, errors of the formaldehyde concentration value measured by the formaldehyde sensor in different environments can be reduced, thereby improving the measurement accuracy of the formaldehyde sensor. For example, the formaldehyde concentration compensation value of the formaldehyde sensor is calculated according to the temperature information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different temperature environments. In another example, the formaldehyde concentration compensation value of the formaldehyde sensor is calculated according to the humidity information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different humidity environments. In yet another example, the formaldehyde concentration compensation value of the formaldehyde sensor is calculated according to the temperature and humidity information of the surrounding environment, and the initial formaldehyde concentration value measured by the formaldehyde sensor is corrected by using the formaldehyde concentration compensation value, so as to reduce the errors of the formaldehyde concentration value measured by the formaldehyde sensor in different temperature and humidity environments.

Referring to FIG. 2, some embodiments of the present invention also provide a formaldehyde concentration measurement method. Based on the embodiment shown in FIG. 1, the step S103, determining the formaldehyde concentration compensation value of the formaldehyde sensor according to the temperature information and/or the humidity information, further includes the following steps.

At step S201, a first difference between the temperature value and a set temperature threshold is calculated, and/or a second difference between the humidity value and a set humidity threshold is calculated.

In some embodiments of the present invention, the set temperature threshold includes but is not limited to 25 degrees, and the set humidity threshold includes but is not limited to 50%. For example, in an environment where the temperature value *a* is 25 degrees, the formaldehyde concentration in the air measured by the formaldehyde sensor is 1.2 mg/m³, i.e., the initial formaldehyde concentration value A measured in the environment where the temperature value *a* is 25 degrees is 1.2 mg/m³. The temperature threshold is subtracted from the temperature value *a* (25 degrees - 25 degrees), and the difference between the temperature value *a* and the temperature threshold is 0, then the formaldehyde concentration compensation value corresponding to the difference is also 0. Thus, there is no need to correct the initial formaldehyde concentration value A, and the initial formaldehyde concentration value A is the target formaldehyde concentration value of the formaldehyde sensor.

In another example, in an environment where the temperature value b is 6 degrees, the formaldehyde concentration in the air measured by the formaldehyde sensor is 1 mg/m³, i.e., the initial formaldehyde concentration value B measured in the environment where the temperature value b is 6 degrees is 1 mg/m³. The temperature threshold is subtracted from the temperature value b (6 degrees - 25 degrees), and the difference between the temperature value b and the temperature threshold is obtained as -19 degrees, then the formaldehyde concentration compensation value corresponding to the difference (-19 degrees) is 0.2 mg/m³. The formaldehyde concentration compensation value (0.2 mg/m³) and the initial formaldehyde concentration value B (1 mg/m³) are added together to obtain the target formaldehyde concentration value (1.2 mg/m³).

At step S202, the formaldehyde concentration compensation value of the formaldehyde sensor is determined according to the first difference and/or the second difference.

In some embodiments of the present invention, the step S202 of determining the formaldehyde concentration compensation value of the formaldehyde sensor according to the first difference and/or the second difference includes steps of:
determining a first formaldehyde concentration compensation value of the formaldehyde sensor corresponding to the first difference between the temperature value and the set temperature threshold, according to a preset corresponding relationship between the first difference, between the temperature value and the set temperature threshold, and the formaldehyde concentration compensation value of the formaldehyde sensor; and/or
determining a second formaldehyde concentration compensation value of the formaldehyde sensor corresponding to the second difference between the humidity value and the set humidity threshold, according to a preset corresponding relationship between the second difference, between the humidity value and the set humidity threshold, and the formaldehyde concentration compensation value of the formaldehyde sensor; and
setting the first formaldehyde concentration compensation value, or the second formaldehyde concentration compensation value, or a sum of the first formaldehyde concentration compensation value and the second formaldehyde concentration compensation value, as the formaldehyde concentration compensation value of the formaldehyde sensor.

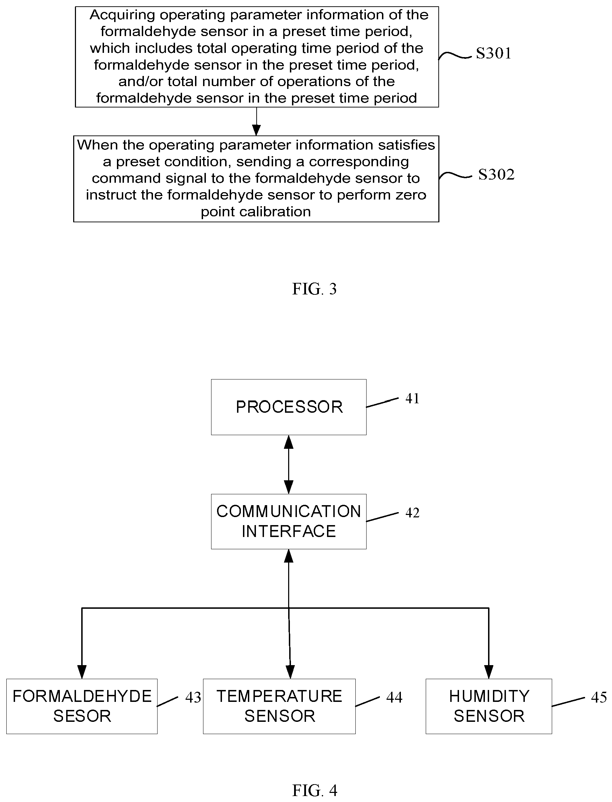

There is a problem that the accuracy of the measured formaldehyde concentration becomes relatively low when the formaldehyde sensor has been operated for a long time. Referring to FIG. 3, some embodiments of the present invention also provide a formaldehyde concentration measurement method. Based on the embodiment shown in FIG. 1, the method further includes the following steps.

At step S301, operating parameter information of the formaldehyde sensor in a preset time period is acquired, wherein the operating parameter information includes: total operating time period of the formaldehyde sensor in the preset time period, and/or total number of operations of the formaldehyde sensor in the preset time period.

In some embodiments of the present invention, the operating parameter information is the information used to describe the operating conditions of the formaldehyde sensor. The operating parameter information can be the total operating time period of the formaldehyde sensor in a preset time period, or the operating parameter information can be the total number of operations of the formaldehyde sensor in a preset time period, or the operating parameter information can include the total operating time period and the total number of operations of the formaldehyde sensor in a preset time period.

Under different formaldehyde concentrations, the total operating time periods of the formaldehyde sensor in the preset time period are different, and the total numbers of operations of the formaldehyde sensor in the preset time period are also different. The higher the formaldehyde concentration, the longer the total operating time period of the formaldehyde sensor in the preset time period, similarly, the larger the total number of operations of the formaldehyde sensor in the preset time period.

For example, in the case where the preset time period is set to 1 hour, when the formaldehyde concentration > a high concentration value N1, the total operating time period of the formaldehyde sensor within 1 hour is recorded as T1 hours;
When medium concentration N2 < the formaldehyde concentration < high concentration N1, the total operating time period of the formaldehyde sensor within 1 hour is recorded as T2 hours;
When low concentration N3 < the formaldehyde concentration < medium concentration N2, the total operating time period of the formaldehyde sensor within 1 hour is recorded as T3 hours;
When 0 <the formaldehyde concentration<low concentration N3, the total operating time period of the formaldehyde sensor within 1 hour is recorded as T4 hours;
Then the relationship among the total operating time periods is: T4<T3<T2<T1.

For example, in the case where the preset time period is set to 1 hour, when the formaldehyde concentration > high concentration value N1, the total number of operations of the formaldehyde sensor within 1 hour is recorded as a1 times;
When medium concentration N2 < the formaldehyde concentration < high concentration N1, the total number of operations of the formaldehyde sensor within 1 hour is recorded as a2 times;
When low concentration N3 < the formaldehyde concentration < medium concentration N2, the total number of operations of the formaldehyde sensor within 1 hour is recorded as a3 times;
When 0 < the formaldehyde concentration<low concentration N3, the total number of operations of the formaldehyde sensor within 1 hour is recorded as a4 times;
Then the relationship among total number of operations is: a4<a3<a2<a1.

At step S302, when the operating parameter information satisfies a preset condition, a corresponding command signal is sent to the formaldehyde sensor in order to instruct the formaldehyde sensor to perform zero point calibration.

In other embodiments of the present invention, the step S302, sending the corresponding command signal to the formaldehyde sensor in order to instruct the formaldehyde sensor to perform the zero point calibration, when the operating parameter information satisfies the preset condition, includes steps of:
comparing the total operating time period of the formaldehyde sensor in a preset time period with a set total operating time period threshold to obtain a first comparison result;
   and/or
comparing the total number of operations of the formaldehyde sensor in a preset time period with a set total number threshold of operations to obtain a second comparison result; and
determining that the operating parameter information satisfies the preset condition when the first comparison result is: the total operating time period of the formaldehyde sensor in the preset time period is greater than the set total operating time period threshold, and/or the second comparison result is: the total number of operations of the formaldehyde sensor in the preset time period is greater than the set total number threshold of operations.

In some embodiments of the present invention, the total operating time period threshold can be set to 30 minutes, and the total number threshold of operations can be set to 15 times, which are not limited in the embodiments of the present invention.

In some embodiments of the present invention, by counting the total operating time period and/or the total number of operations of the formaldehyde sensor in a preset time period, and sending command signals to the formaldehyde sensor periodically by a processor to allow the formaldehyde sensor to re-calibrate the zero point, the self-calibration of the formaldehyde sensor can be realized, the problem of zero point drift caused by the long-term operation of the formaldehyde sensor is solved, and the accuracy of the formaldehyde concentration measured by the formaldehyde sensor is improved.

Referring to FIG. 4, some embodiments of the present invention provide a formaldehyde concentration measurement apparatus including a processor 41, a communication interface 42, a formaldehyde sensor 43, and a temperature sensor 44 and/or a humidity sensor 45. The formaldehyde sensor 43 and the temperature sensor 44 and/or the humidity sensor 45 are electrically connected to the processor 41 via the communication interface 42.

The formaldehyde sensor 43 is configured to measure the formaldehyde concentration to obtain an initial formaldehyde concentration value, and send the initial formaldehyde concentration value to the processor 41.

The temperature sensor 44 is configured to collect temperature information of the environment surrounding the formaldehyde sensor 43, and send the temperature information to the processor 41.

The temperature sensor 45 is configured to collect humidity information of the environment surrounding the formaldehyde sensor 43, and send the humidity information to the processor 41.

The processor 41 is configured to determine a formaldehyde concentration compensation value of the formaldehyde sensor 43 according to the received temperature information and/or humidity information, and to determine a target formaldehyde concentration value of the formaldehyde sensor 43 according to the formaldehyde concentration compensation value and the received initial formaldehyde concentration value.

In other embodiments of the present invention, the temperature information is a temperature value, the humidity information is a humidity value, and the processor 41 includes:
a difference calculation module configured to calculate a first difference between the temperature value and a set temperature threshold, and/or calculating a second difference between the humidity value and a set humidity threshold; and
a compensation value determination module configured to determine the formaldehyde concentration compensation value of the formaldehyde sensor 43 according to the first difference and/or the second difference calculated by the difference calculation module.

In other embodiments of the present invention, the compensation value determination module includes:
a first compensation value determination unit configured to determine the first formaldehyde concentration compensation value of the formaldehyde sensor 43 corresponding to the first difference between the temperature value and the set temperature threshold, according to a preset corresponding relationship between the first difference, between the temperature value and the set temperature threshold, and the formaldehyde concentration compensation value of the formaldehyde sensor 43; and/or
a second compensation value determination unit configured to determine the second formaldehyde concentration compensation value of the formaldehyde sensor 43 corresponding to the second difference between the humidity value and the set humidity threshold, according to a preset corresponding relationship between the second difference, between the humidity value and the set humidity threshold, and the formaldehyde concentration compensation value of the formaldehyde sensor 43; and
a formaldehyde concentration compensation value determination unit configured to set the first formaldehyde concentration compensation value determined by the first compensation value determination unit, or the second formaldehyde concentration compensation value determined by the second compensation value determination unit, or a sum of the first formaldehyde concentration compensation value determined by the first compensation value determination unit and the second formaldehyde concentration compensation value determined by the second compensation value determination unit, as the formaldehyde concentration compensation value of the formaldehyde sensor 43.

In other embodiments of the present invention, the processor 41 further includes
a parameter information acquisition module configured to acquire operating parameter information of the formaldehyde sensor 43 in a preset time period. The operating parameter information includes: total operating time period of the formaldehyde sensor 43 in the preset time period, and/or total number of operations of the formaldehyde sensor 43 in the preset time period.

The processor 41 sends a corresponding command signal to the formaldehyde sensor 43 in order to instruct the formaldehyde sensor 43 to perform zero point calibration, on a condition that the operating parameter information acquired by the parameter information acquisition module satisfies a preset condition.

In other embodiments of the present invention, the processor 41 further includes:
a first comparison module configured to compare the total operating time period of the formaldehyde sensor 43 in the preset time period with a set total operating time period threshold to obtain a first comparison result; and/or,
a second comparison module configured to compare the total number of operations of the formaldehyde sensor 43 in the preset time period with a set total number threshold of operations to obtain a second comparison result.

The processor 41 determines that the operating parameter information acquired by the parameter information acquisition module satisfies the preset condition, on a condition that the first comparison result obtained by the first comparison module is: the total operating time period of the formaldehyde sensor 43 in the preset time period is greater than the set total operating time period threshold, and/or on a condition that the second comparison result obtained by the second comparison module is: the total number of operations of the formaldehyde sensor 43 in the preset time period is greater than the set total number threshold of operations.

Some embodiments of the present invention also provide an air purifier, including the formaldehyde concentration measurement apparatus as shown in FIG. 4.

For the convenience of description, the above apparatus is described by illustrating various units that are divided according to their functions. Certainly, when implementing the present invention, the functions of the units can be implemented in the same one or more software and/or hardware components.

The various embodiments in this specification are described in a progressive manner, and the same or similar parts between the various embodiments can be referred to each other. Each embodiment focuses on the differences from other embodiments. In particular, for the apparatus or system embodiment, since it is basically similar to the method embodiment, the description is relatively simple, and the relevant part can refer to the part of the description of the method embodiment. The above-described apparatus and system embodiments are merely illustrative, wherein the units described as separate components may or may not be physically separated, and the components shown as units may or may not be physical units, i.e., they may be located in one place, or may be distributed to multiple network units. Some or all of the modules can be selected according to actual needs to achieve the objectives of the solutions of the embodiments. The present invention can be understood and implemented without creative work by those of ordinary skill in the art.

It should be noted that, relational terms such as "first" and "second" herein are only used to distinguish one entity or operation from another entity or operation, and do not necessarily require or imply that there is any such actual relationship or sequence between entities or operations. Moreover, the terms "include", "comprise" or any other variants thereof are intended to cover non-exclusive inclusion, so that a process, method, product or device including a series of elements not only includes those elements, but also includes those that are not explicitly listed, or the inherent elements of this process, method, product or device. Without further limitations, the element defined by the expression "including a..." does not exclude the existence of other identical elements in the process, method, product or device that includes the element.

The above are only specific embodiments of the present invention, which enables those skilled in the art to understand or implement the present invention. Various modifications to these embodiments will be apparent to those skilled in the art, and the general principles defined herein can be implemented in other embodiments without departing from the scope of the present invention.

## Claims

1. A formaldehyde concentration measurement method, comprising:
determining an initial formaldehyde concentration value measured by a formaldehyde sensor (43);
acquiring temperature information of an environment surrounding the formaldehyde sensor (43), and determining a formaldehyde concentration compensation value of the formaldehyde sensor (43) according to the temperature information; or acquiring humidity information of the environment surrounding the formaldehyde sensor (43), and determining the formaldehyde concentration compensation value of the formaldehyde sensor (43) according to the humidity information; or acquiring the temperature information and the humidity information of the environment surrounding the formaldehyde sensor (43), and determining the formaldehyde concentration compensation value of the formaldehyde sensor (43) according to the temperature information and the humidity information; and
determining a target formaldehyde concentration value of the formaldehyde sensor (43) according to the formaldehyde concentration compensation value and the initial formaldehyde concentration value;
**characterized by** further comprising:
acquiring operating parameter information of the formaldehyde sensor (43) in a preset time period, wherein the operating parameter information comprises: total operating time period of the formaldehyde sensor (43) in the preset time period, or total number of operations of the formaldehyde sensor (43) in the preset time period, or the total operating time period of the formaldehyde sensor (43) in the preset time period and the total number of operations of the formaldehyde sensor (43) in the preset time period; and
sending a corresponding command signal to the formaldehyde sensor (43) in order to instruct the formaldehyde sensor (43) to perform zero point calibration when the operating parameter information satisfies a preset condition; and
re-calibrating the zero point by the formaldehyde sensor in response to the corresponding command signal;
wherein the sending the corresponding command signal to the formaldehyde sensor (43) in order to instruct the formaldehyde sensor (43) to perform the zero point calibration, when the operating parameter information satisfies the preset condition, comprises:
when the operating parameter information of the formaldehyde sensor (43) in the preset time period is the total operating time period of the formaldehyde sensor (43) in the preset time period, comparing the total operating time period of the formaldehyde sensor (43) in the preset time period with a set total operating time period threshold to obtain a first comparison result; determining that the operating parameter information satisfies the preset condition when the first comparison result is that the total operating time period of the formaldehyde sensor (43) in the preset time period is greater than the set total operating time period threshold;
when the operating parameter information of the formaldehyde sensor (43) in the preset time period is the total number of operations of the formaldehyde sensor (43) in the preset time period, comparing the total number of operations of the formaldehyde sensor (43) in the preset time period with a set total number threshold of operations to obtain a second comparison result; determining that the operating parameter information satisfies the preset condition when the second comparison result is that the total number of operations of the formaldehyde sensor (43) in the preset time period is greater than the set total number threshold of operations;
when the operating parameter information of the formaldehyde sensor (43) in the preset time period comprises the total operating time period of the formaldehyde sensor (43) in the preset time period and the total number of operations of the formaldehyde sensor (43) in the preset time period, comparing the total operating time period of the formaldehyde sensor (43) in the preset time period with the set total operating time threshold to obtain the first comparison result, and comparing the total number of operations of the formaldehyde sensor (43) in the preset time period with the set total number threshold of operations to obtain the second comparison result; and determining that the operating parameter information satisfies the preset condition when the first comparison result is that the total operating time period of the formaldehyde sensor (43) in the preset time period is greater than the set total operating time period threshold, and the second comparison result is that the total number of operations of the formaldehyde sensor (43) in the preset time period is greater than the set total number threshold of operations.

2. The method according to claim 1, wherein the temperature information is a temperature value, and the humidity information is a humidity value;
the determining the formaldehyde concentration compensation value of the formaldehyde sensor (43) according to the temperature information comprises: calculating a first difference between the temperature value and a set temperature threshold, and determining the formaldehyde concentration compensation value of the formaldehyde sensor (43) according to the first difference;
the determining the formaldehyde concentration compensation value of the formaldehyde sensor (43) according to the humidity information comprises: calculating a second difference between the humidity value and a set humidity threshold, and determining the formaldehyde concentration compensation value of the formaldehyde sensor (43) according to the second difference; and
the determining the formaldehyde concentration compensation value of the formaldehyde sensor (43) according to the temperature information and the humidity information comprises: calculating the first difference between the temperature value and the set temperature threshold and the second difference between the humidity value and the set humidity threshold; and determining the formaldehyde concentration compensation value of the formaldehyde sensor (43) according to the first difference and the second difference.

3. The method according to claim 2, wherein
the determining the formaldehyde concentration compensation value of the formaldehyde sensor (43) according to the first difference comprises: determining a first formaldehyde concentration compensation value of the formaldehyde sensor (43) corresponding to the first difference between the temperature value and the set temperature threshold, according to a preset corresponding relationship between the first difference and the formaldehyde concentration compensation value of the formaldehyde sensor (43); and setting the first formaldehyde concentration compensation value as the formaldehyde concentration compensation value of the formaldehyde sensor (43);
the determining the formaldehyde concentration compensation value of the formaldehyde sensor (43) according to the second difference comprises: determining a second formaldehyde concentration compensation value of the formaldehyde sensor (43) corresponding to the second difference between the humidity value and the set humidity threshold, according to a preset corresponding relationship between the second difference and the formaldehyde concentration compensation value of the formaldehyde sensor (43); and setting the second formaldehyde concentration compensation value as the formaldehyde concentration compensation value of the formaldehyde sensor (43);
the determining the formaldehyde concentration compensation value of the formaldehyde sensor (43) according to the first difference and the second difference comprises: determining the first formaldehyde concentration compensation value of the formaldehyde sensor (43) corresponding to the first difference between the temperature value and the set temperature threshold, according to the preset corresponding relationship between the first difference and the formaldehyde concentration compensation value of the formaldehyde sensor (43); determining the second formaldehyde concentration compensation value of the formaldehyde sensor (43) corresponding to the second difference between the humidity value and the set humidity threshold, according to the preset corresponding relationship between the second difference and the formaldehyde concentration compensation value of the formaldehyde sensor (43); and setting a sum of the first formaldehyde concentration compensation value and the second formaldehyde concentration compensation value as the formaldehyde concentration compensation value of the formaldehyde sensor (43).

4. A formaldehyde concentration measurement apparatus, comprising:
a processor (41);
a communication interface (42);
a formaldehyde sensor (43) electrically connected to the processor (41) via the communication interface;
a temperature sensor (44), or a humidity sensor (45), or the temperature sensor (44) and the humidity sensor (45), electrically connected to the processor (41) via the communication interface (42),
wherein the formaldehyde sensor (43) is configured to measure a concentration of formaldehyde to obtain an initial formaldehyde concentration value, and to send the initial formaldehyde concentration value to the processor (41);
the temperature sensor (44) is configured to collect temperature information of an environment surrounding the formaldehyde sensor (43), and to send the temperature information to the processor (41);
the temperature sensor (44) is configured to collect humidity information of the environment surrounding the formaldehyde sensor (43), and to send the humidity information to the processor (41);
when the apparatus comprises the temperature sensor (44), the processor (41) is configured to determine a formaldehyde concentration compensation value of the formaldehyde sensor (43) according to the temperature information that is received;
when the apparatus comprises the humidity sensor (45), the processor (41) is configured to determine the formaldehyde concentration compensation value of the formaldehyde sensor (43) according to the humidity information that is received;
when the apparatus comprises the temperature sensor (44) and the humidity sensor (45), the processor (41) is configured to determine the formaldehyde concentration compensation value of the formaldehyde sensor (43) according to the temperature information and humidity information that are received; and
the processor (41) is configured to determine a target formaldehyde concentration value of the formaldehyde sensor (43) according to the formaldehyde concentration compensation value and the initial formaldehyde concentration value that is received;
**characterized in that** the formaldehyde concentration measurement apparatus further comprises:
a parameter information acquisition module configured to acquire operating parameter information of the formaldehyde sensor (43) in a preset time period, wherein the operating parameter information comprises: total operating time period of the formaldehyde sensor (43) in the preset time period, or total number of operations of the formaldehyde sensor (43) in the preset time period, or the total operating time period of the formaldehyde sensor (43) in the preset time period and the total number of operations of the formaldehyde sensor (43) in the preset time period;
the processor (41) is configured to send a corresponding command signal to the formaldehyde sensor (43) in order to instruct the formaldehyde sensor (43) to perform zero point calibration when the operating parameter information acquired by the parameter information acquisition module satisfies a preset condition; and
the formaldehyde sensor (43) is configured to perform zero point calibration in response to the corresponding command signal;
wherein the processor (41) further comprises a first comparison module, or a second comparison module, or the first comparison module and the second comparison module;
when the operating parameter information comprises the total operating time period of the formaldehyde sensor (43) in the preset time period, the processor (41) further comprises the first comparison module; when the operating parameter information comprises the total number of operations of the formaldehyde sensor (43) in the preset time period, the processor (41) further comprises the second comparison module; when the operating parameter information comprises the total operating time period of the formaldehyde sensor (43) in the preset time period and the total number of operations of the formaldehyde sensor (43) in the preset time period, the processor (41) further comprises the first comparison module and the second comparison module;
the first comparison module is configured to compare the total operating time period of the formaldehyde sensor (43) in the preset time period with a set total operating time period threshold to obtain a first comparison result;
the second comparison module is configured to compare the total number of operations of the formaldehyde sensor (43) in the preset time period with a set total number threshold of operations to obtain a second comparison result;
when the processor (41) further comprises the first comparison module, and when the first comparison result obtained by the first comparison module is that the total operating time period of the formaldehyde sensor (43) in the preset time period is greater than the set total operating time period threshold, the processor (41) determines that the operating parameter information acquired by the parameter information acquisition module satisfies the preset condition;
when the processor (41) further comprises the second comparison module, and when the second comparison result obtained by the second comparison module is that the total number of operations of the formaldehyde sensor (43) in the preset time period is greater than the set total number threshold of operations, the processor (41) determines that the operating parameter information acquired by the parameter information acquisition module satisfies the preset condition;
when the processor (41) further comprises the first comparison module and the second comparison module, and when the first comparison result obtained by the first comparison module is that the total operating time period of the formaldehyde sensor (43) in the preset time period is greater than the set total operating time period threshold, and the second comparison result obtained by the second comparison module is that the total number of operations of the formaldehyde sensor (43) in the preset time period is greater than the set total number threshold of operations, the processor (41) determines that the operating parameter information acquired by the parameter information acquisition module satisfies the preset condition.

5. The apparatus according to claim 4, wherein the temperature information is a temperature value, the humidity information is a humidity value, and the processor (41) comprises a difference calculation module and a compensation value determination module;
when the apparatus comprises the temperature sensor (44), the difference calculation module is configured to calculate a first difference between the temperature value and a set temperature threshold, the compensation value determination module is configured to determine the formaldehyde concentration compensation value of the formaldehyde sensor (43) according to the first difference calculated by the difference calculation module;
when the apparatus comprises the humidity sensor (45), the difference calculation module is configured to calculate a second difference between the humidity value and a set humidity threshold, the compensation value determination module is configured to determine the formaldehyde concentration compensation value of the formaldehyde sensor (43) according to the second difference calculated by the difference calculation module;
when the apparatus comprises the temperature sensor (44) and the humidity sensor (45), the difference calculation module is configured to calculate the first difference between the temperature value and the set temperature threshold and the second difference between the humidity value and the set humidity threshold, the compensation value determination module is configured to determine the formaldehyde concentration compensation value of the formaldehyde sensor (43) according to the first difference and the second difference calculated by the difference calculation module.

6. The apparatus according to claim 5, wherein the compensation value determination module comprises:
a first compensation determination unit, or a second compensation determination unit, or the first compensation determination unit and the second compensation determination unit; and
a formaldehyde concentration compensation value determination unit;
when the difference calculation module is configured to calculate the first difference between the temperature value and the set temperature threshold, the compensation value determination module comprises the first compensation determination unit;
when the difference calculation module is configured to calculate the second difference between the humidity value and the set humidity threshold, the compensation value determination module comprises the second compensation determination unit;
when the difference calculation module is configured to calculate the first difference between the temperature value and the set temperature threshold and the second difference between the humidity value and the set humidity threshold, the compensation value determination module comprises the first compensation determination unit and the second compensation determination unit;
the first compensation value determination unit is configured to determine a first formaldehyde concentration compensation value of the formaldehyde sensor (43) corresponding to the first difference between the temperature value and the set temperature threshold, according to a preset corresponding relationship between the first difference and the formaldehyde concentration compensation value of the formaldehyde sensor (43);
the second compensation value determination unit is configured to determine a second formaldehyde concentration compensation value of the formaldehyde sensor (43) corresponding to the second difference between the humidity value and the set humidity threshold, according to a preset corresponding relationship between the second difference and the formaldehyde concentration compensation value of the formaldehyde sensor (43);
when the difference calculation module is configured to calculate the first difference between the temperature value and the set temperature threshold, the formaldehyde concentration compensation value determination unit is configured to set the first formaldehyde concentration compensation value determined by the first compensation value determination unit as the formaldehyde concentration compensation value of the formaldehyde sensor (43);
when the difference calculation module is configured to calculate the second difference between the humidity value and the set humidity threshold, the formaldehyde concentration compensation value determination unit is configured to set the second formaldehyde concentration compensation value determined by the second compensation value determination unit as the formaldehyde concentration compensation value of the formaldehyde sensor (43); and
when the difference calculation module is configured to calculate the first difference between the temperature value and the set temperature threshold and the second difference between the humidity value and the set humidity threshold, the formaldehyde concentration compensation value determination unit is configured to set a sum of the first formaldehyde concentration compensation value determined by the first compensation value determination unit and the second formaldehyde concentration compensation value determined by the second compensation value determination unit, as the formaldehyde concentration compensation value of the formaldehyde sensor (43).

7. An air purifier, comprising the formaldehyde concentration measurement apparatus according to any one of claims 4 to 6.

## Patentansprüche

1. Verfahren zur Messung der Formaldehydkonzentration, das Folgendes umfasst:
Bestimmen eines Anfangswertes der Formaldehydkonzentration, gemessen mit einem Formaldehydsensor (43);
Erfassen von Temperaturinformationen einer den Formaldehydsensor (43) umgebenden Umgebung und Bestimmen eines Formaldehydkonzentrations-Kompensationswertes des Formaldehydsensors (43) gemäß den Temperaturinformationen; oder Erfassen von Feuchtigkeitsinformationen der den Formaldehydsensor (43) umgebenden Umgebung und Bestimmen des Formaldehydkonzentrations-Kompensationswertes des Formaldehydsensors (43) gemäß den Feuchtigkeitsinformationen; oder Erfassen der Temperaturinformation und der Feuchtigkeitsinformation der den Formaldehydsensor (43) umgebenden Umgebung und Bestimmen des Formaldehydkonzentrations-Kompensationswertes des Formaldehydsensors (43) gemäß der Temperaturinformation und der Feuchtigkeitsinformation; und
Bestimmen eines Zielwertes für die Formaldehydkonzentration des Formaldehydsensors (43) entsprechend dem Formaldehydkonzentrations-Kompensationswert und dem Formaldehydkonzentrations-Anfangswert;
**dadurch gekennzeichnet, dass** es ferner Folgendes umfasst:
Erfassen von Betriebsparameterinformationen des Formaldehydsensors (43) in einer voreingestellten Zeitperiode, wobei die Betriebsparameterinformationen Folgendes umfassen: Gesamtbetriebszeitperiode des Formaldehydsensors (43) in der voreingestellten Zeitperiode oder Gesamtzahl von Operationen des Formaldehydsensors (43) in der voreingestellten Zeitperiode oder die Gesamtbetriebszeitperiode des Formaldehydsensors (43) in der voreingestellten Zeitperiode und die Gesamtzahl von Operationen des Formaldehydsensors (43) in der voreingestellten Zeitperiode; und
Senden eines entsprechenden Befehlssignals an den Formaldehydsensor (43), um den Formaldehydsensor (43) anzuweisen, eine Nullpunktkalibrierung durchzuführen, wenn die Betriebsparameterinformation eine vorgegebene Bedingung erfüllt; und
Neukalibrierung des Nullpunkts durch den Formaldehydsensor in Reaktion auf das entsprechende Befehlssignal;
wobei das Senden des entsprechenden Befehlssignals an den Formaldehydsensor (43), um den Formaldehydsensor (43) anzuweisen, die Nullpunktkalibrierung durchzuführen, wenn die Betriebsparameterinformation die voreingestellte Bedingung erfüllt, Folgendes umfasst:
wenn die Betriebsparameterinformation des Formaldehydsensors (43) in der voreingestellten Zeitperiode die Gesamtbetriebszeitperiode des Formaldehydsensors (43) in der voreingestellten Zeitperiode ist, Vergleichen der Gesamtbetriebszeitperiode des Formaldehydsensors (43) in der voreingestellten Zeitperiode mit einem eingestellten Gesamtbetriebszeitperioden-Schwellenwert, um ein erstes Vergleichsergebnis zu erhalten; Bestimmen, dass die Betriebsparameterinformation die voreingestellte Bedingung erfüllt, wenn das erste Vergleichsergebnis ist, dass die Gesamtbetriebszeitperiode des Formaldehydsensors (43) in der voreingestellten Zeitperiode größer ist als der eingestellte Gesamtbetriebszeitperioden-Schwellenwert;
wenn die Betriebsparameterinformation des Formaldehydsensors (43) in der voreingestellten Zeitperiode die Gesamtzahl von Operationen des Formaldehydsensors (43) in der voreingestellten Zeitperiode ist, Vergleichen der Gesamtzahl von Operationen des Formaldehydsensors (43) in der voreingestellten Zeitperiode mit einem eingestellten Gesamtzahlschwellenwert von Operationen, um ein zweites Vergleichsergebnis zu erhalten; Bestimmen, dass die Betriebsparameterinformation die voreingestellte Bedingung erfüllt, wenn das zweite Vergleichsergebnis ist, dass die Gesamtzahl von Operationen des Formaldehydsensors (43) in der voreingestellten Zeitperiode größer ist als der eingestellte Gesamtzahlschwellenwert von Operationen;
wenn die Betriebsparameterinformation des Formaldehydsensors (43) in der voreingestellten Zeitperiode die Gesamtbetriebszeitperiode des Formaldehydsensors (43) in der voreingestellten Zeitperiode und die Gesamtzahl der Operationen des Formaldehydsensors (43) in der voreingestellten Zeitperiode umfasst, Vergleichen der Gesamtbetriebszeitperiode des Formaldehydsensors (43) in der voreingestellten Zeitperiode mit dem eingestellten Gesamtbetriebszeitperioden-Schwellenwert, um das erste Vergleichsergebnis zu erhalten, und Vergleichen der Gesamtzahl von Operationen des Formaldehydsensors (43) in der voreingestellten Zeitperiode mit dem eingestellten Gesamtanzahl-Schwellenwert von Operationen, um das zweite Vergleichsergebnis zu erhalten; und Bestimmen, dass die Betriebsparameterinformation die voreingestellte Bedingung erfüllt, wenn das erste Vergleichsergebnis ist, dass die Gesamtbetriebszeitperiode des Formaldehydsensors (43) in der voreingestellten Zeitperiode größer ist als der eingestellte Gesamtbetriebszeitperioden-Schwellenwert, und das zweite Vergleichsergebnis ist, dass die Gesamtzahl der Operationen des Formaldehydsensors (43) in der voreingestellten Zeitperiode größer ist als der eingestellte Gesamtanzahl-Schwellenwert der Operationen.

2. Verfahren nach Anspruch 1, wobei die Temperaturinformation ein Temperaturwert ist und die Feuchtigkeitsinformation ein Feuchtigkeitswert ist;
das Bestimmen des Formaldehydkonzentrations-Kompensationswertes des Formaldehydsensors (43) gemäß der Temperaturinformation Folgendes umfasst: Berechnen einer ersten Differenz zwischen dem Temperaturwert und einem eingestellten Temperaturschwellenwert, und Bestimmen des Formaldehydkonzentrations-Kompensationswertes des Formaldehydsensors (43) gemäß der ersten Differenz;
das Bestimmen des Formaldehydkonzentrations-Kompensationswertes des Formaldehydsensors (43) gemäß der Feuchtigkeitsinformation Folgendes umfasst:
Berechnen einer zweiten Differenz zwischen dem Feuchtigkeitswert und einem eingestellten Feuchtigkeitsschwellenwert, und Bestimmen des Formaldehydkonzentrations-Kompensationswertes des Formaldehydsensors (43) gemäß der zweiten Differenz; und
das Bestimmen des Formaldehydkonzentrations-Kompensationswertes des Formaldehydsensors (43) gemäß der Temperaturinformation und der Feuchtigkeitsinformation Folgendes umfasst: Berechnen der ersten Differenz zwischen dem Temperaturwert und dem eingestellten Temperaturschwellenwert und der zweiten Differenz zwischen dem Feuchtigkeitswert und dem eingestellten Feuchtigkeitsschwellenwert; und Bestimmen des Formaldehydkonzentrations-Kompensationswertes des Formaldehydsensors (43) gemäß der ersten Differenz und der zweiten Differenz.

3. Verfahren nach Anspruch 2, wobei
das Bestimmen des Formaldehydkonzentrations-Kompensationswertes des Formaldehydsensors (43) gemäß der ersten Differenz Folgendes umfasst: Bestimmen eines ersten Formaldehydkonzentrations-Kompensationswertes des Formaldehydsensors (43), der der ersten Differenz zwischen dem Temperaturwert und dem eingestellten Temperaturschwellenwert entspricht, gemäß einer voreingestellten entsprechenden Beziehung zwischen der ersten Differenz und dem Formaldehydkonzentrations-Kompensationswert des Formaldehydsensors (43); und Einstellen des ersten Formaldehydkonzentrations-Kompensationswertes als den Formaldehydkonzentrations-Kompensationswert des Formaldehydsensors (43);
das Bestimmen des Formaldehydkonzentrations-Kompensationswertes des Formaldehydsensors (43) gemäß der zweiten Differenz Folgendes umfasst: Bestimmen eines zweiten Formaldehydkonzentrations-Kompensationswertes des Formaldehydsensors (43), der der zweiten Differenz zwischen dem Feuchtigkeitswert und dem eingestellten Feuchtigkeitsschwellenwert entspricht, gemäß einer voreingestellten entsprechenden Beziehung zwischen der zweiten Differenz und dem Formaldehydkonzentrations-Kompensationswert des Formaldehydsensors (43); und Einstellen des zweiten Formaldehydkonzentrations-Kompensationswertes als den Formaldehydkonzentrations-Kompensationswert des Formaldehydsensors (43);
das Bestimmen des Formaldehydkonzentrations-Kompensationswertes des Formaldehydsensors (43) entsprechend der ersten Differenz und der zweiten Differenz Folgendes umfasst: Bestimmen des ersten Formaldehydkonzentrations-Kompensationswertes des Formaldehydsensors (43) entsprechend der ersten Differenz zwischen dem Temperaturwert und dem eingestellten Temperaturschwellenwert, gemäß der voreingestellten entsprechenden Beziehung zwischen der ersten Differenz und dem Formaldehydkonzentrations-Kompensationswert des Formaldehydsensors (43); Bestimmen des zweiten Formaldehydkonzentrations-Kompensationswertes des Formaldehydsensors (43) entsprechend der zweiten Differenz zwischen dem Feuchtigkeitswert und dem eingestellten Feuchtigkeitsschwellenwert, gemäß der voreingestellten entsprechenden Beziehung zwischen der zweiten Differenz und dem Formaldehydkonzentrations-Kompensationswert des Formaldehydsensors (43); und Festlegen einer Summe des ersten Formaldehydkonzentrations-Kompensationswertes und des zweiten Formaldehydkonzentrations-Kompensationswertes als den Formaldehydkonzentrations-Kompensationswert des Formaldehydsensors (43).

4. Vorrichtung zur Messung der Formaldehydkonzentration, die Folgendes umfasst:
einen Prozessor (41);
eine Kommunikationsschnittstelle (42);
einen Formaldehydsensor (43), der über die Kommunikationsschnittstelle elektrisch mit dem Prozessor (41) verbunden ist;
einen Temperatursensor (44) oder einen Feuchtigkeitssensor (45) oder den Temperatursensor (44) und den Feuchtigkeitssensor (45), die über die Kommunikationsschnittstelle (42) elektrisch mit dem Prozessor (41) verbunden sind,
wobei der Formaldehydsensor (43) ausgebildet ist, um eine Formaldehydkonzentration zu messen, um einen Anfangswert der Formaldehydkonzentration zu erhalten, und um den Anfangswert der Formaldehydkonzentration an den Prozessor (41) zu senden;
der Temperatursensor (44) ausgebildet ist, um Temperaturinformationen einer Umgebung zu sammeln, die den Formaldehydsensor (43) umgibt, und um die Temperaturinformationen an den Prozessor (41) zu senden;
der Temperatursensor (44) ausgebildet ist, um Feuchtigkeitsinformationen der den Formaldehydsensor (43) umgebenden Umgebung zu sammeln und die Feuchtigkeitsinformationen an den Prozessor (41) zu senden;
wenn die Vorrichtung den Temperatursensor (44) umfasst, der Prozessor (41) ausgebildet ist, um einen Formaldehydkonzentrations-Kompensationswert des Formaldehydsensors (43) gemäß der empfangenen Temperaturinformation zu bestimmen;
wenn die Vorrichtung den Feuchtigkeitssensor (45) umfasst, ist der Prozessor (41) ausgebildet, um den Formaldehydkonzentrations-Kompensationswert des Formaldehydsensors (43) gemäß der empfangenen Feuchtigkeitsinformation zu bestimmen;
wenn die Vorrichtung den Temperatursensor (44) und den Feuchtigkeitssensor (45) umfasst, der Prozessor (41) ausgebildet ist, um den Formaldehydkonzentrations-Kompensationswert des Formaldehydsensors (43) gemäß den empfangenen Temperaturinformationen und Feuchtigkeitsinformationen zu bestimmen; und
der Prozessor (41) ausgebildet ist, um einen Zielwert der Formaldehydkonzentration des Formaldehydsensors (43) gemäß dem Formaldehydkonzentrations-Kompensationswert und dem empfangenen Formaldehydkonzentrations-Anfangswert zu bestimmen;
**dadurch gekennzeichnet, dass** die Vorrichtung zur Messung der Formaldehydkonzentration ferner Folgendes umfasst:
ein Parameterinformationserfassungsmodul, das ausgebildet ist, um Betriebsparameterinformationen des Formaldehydsensors (43) in einer voreingestellten Zeitperiode zu erfassen, wobei die Betriebsparameterinformationen Folgendes umfassen: die Gesamtbetriebszeitperiode des Formaldehydsensors (43) in der voreingestellten Zeitperiode, oder die Gesamtzahl der Operationen des Formaldehydsensors (43) in der voreingestellten Zeitperiode, oder die Gesamtbetriebszeitperiode des Formaldehydsensors (43) in der voreingestellten Zeitperiode und die Gesamtzahl der Operationen des Formaldehydsensors (43) in der voreingestellten Zeitperiode;
der Prozessor (41) ausgebildet ist, um ein entsprechendes Befehlssignal an den Formaldehydsensor (43) zu senden, um den Formaldehydsensor (43) anzuweisen, eine Nullpunktkalibrierung durchzuführen, wenn die von dem Parameterinformationserfassungsmodul erfasste Betriebsparameterinformation eine voreingestellte Bedingung erfüllt; und
der Formaldehydsensor (43) ausgebildet ist, um eine Nullpunktkalibrierung in Reaktion auf das entsprechende Befehlssignal durchzuführen;
wobei der Prozessor (41) ferner ein erstes Vergleichsmodul oder ein zweites Vergleichsmodul, oder das erste Vergleichsmodul und das zweite Vergleichsmodul umfasst;
wenn die Betriebsparameterinformation die Gesamtbetriebszeitperiode des Formaldehydsensors (43) in der voreingestellten Zeitperiode umfasst, umfasst der Prozessor (41) ferner das erste Vergleichsmodul; wenn die Betriebsparameterinformation die Gesamtzahl der Operationen des Formaldehydsensors (43) in der voreingestellten Zeitperiode umfasst, umfasst der Prozessor (41) ferner das zweite Vergleichsmodul; wenn die Betriebsparameterinformation die Gesamtbetriebszeitperiode des Formaldehydsensors (43) in der voreingestellten Zeitperiode und die Gesamtzahl der Operationen des Formaldehydsensors (43) in der voreingestellten Zeitperiode umfasst, umfasst der Prozessor (41) ferner das erste Vergleichsmodul und das zweite Vergleichsmodul;
das erste Vergleichsmodul ausgebildet ist, um die Gesamtbetriebszeitperiode des Formaldehydsensors (43) in der voreingestellten Zeitperiode mit einem eingestellten Gesamtbetriebszeitperioden-Schwellenwert zu vergleichen, um ein erstes Vergleichsergebnis zu erhalten;
das zweite Vergleichsmodul ausgebildet ist, um die Gesamtzahl der Operationen des Formaldehydsensors (43) in der voreingestellten Zeitperiode mit einer eingestellten Gesamtzahlschwelle von Operationen zu vergleichen, um ein zweites Vergleichsergebnis zu erhalten;
wenn der Prozessor (41) ferner das erste Vergleichsmodul umfasst, und wenn das von dem ersten Vergleichsmodul erhaltene erste Vergleichsergebnis darin besteht, dass die Gesamtbetriebszeitperiode des Formaldehydsensors (43) in der voreingestellten Zeitperiode größer ist als der eingestellte Schwellenwert für die Gesamtbetriebszeitperiode, bestimmt der Prozessor (41), dass die von dem Parameterinformationserfassungsmodul erfasste Betriebsparameterinformation die voreingestellte Bedingung erfüllt;
wenn der Prozessor (41) ferner das zweite Vergleichsmodul umfasst, und wenn das durch das zweite Vergleichsmodul erhaltene zweite Vergleichsergebnis darin besteht, dass die Gesamtzahl der Operationen des Formaldehydsensors (43) in der voreingestellten Zeitperiode größer ist als der eingestellte Schwellenwert für die Gesamtzahl der Operationen, bestimmt der Prozessor (41), dass die durch das Parameterinformationserfassungsmodul erfasste Betriebsparameterinformation die voreingestellte Bedingung erfüllt;
wenn der Prozessor (41) ferner das erste Vergleichsmodul und das zweite Vergleichsmodul umfasst, und wenn das durch das erste Vergleichsmodul erhaltene erste Vergleichsergebnis darin besteht, dass die Gesamtbetriebszeitperiode des Formaldehydsensors (43) in der voreingestellten Zeitperiode größer ist als der eingestellte Gesamtbetriebszeitperioden-Schwellenwert, und das zweite Vergleichsergebnis, das durch das zweite Vergleichsmodul erhalten wird, ist, dass die Gesamtzahl der Operationen des Formaldehydsensors (43) in der voreingestellten Zeitperiode größer ist als der eingestellte Schwellenwert für die Gesamtzahl der Operationen, bestimmt der Prozessor (41), dass die Betriebsparameterinformation, die durch das Parameterinformationserfassungsmodul betrieben wird, die voreingestellte Bedingung erfüllt.

5. Vorrichtung nach Anspruch 4, wobei die Temperaturinformation ein Temperaturwert ist, die Feuchtigkeitsinformation ein Feuchtigkeitswert ist, und der Prozessor (41) ein Differenzberechnungsmodul und ein Kompensationswert-Bestimmungsmodul umfasst;
wenn die Vorrichtung den Temperatursensor (44) umfasst, das Differenzberechnungsmodul ausgebildet ist, um eine erste Differenz zwischen dem Temperaturwert und einem eingestellten Temperaturschwellenwert zu berechnen, das Kompensationswert-Bestimmungsmodul ausgebildet ist, um den Formaldehydkonzentrations-Kompensationswert des Formaldehydsensors (43) gemäß der von dem Differenzberechnungsmodul berechneten ersten Differenz zu bestimmen;
wenn die Vorrichtung den Feuchtigkeitssensor (45) umfasst, das Differenzberechnungsmodul ausgebildet ist, um eine zweite Differenz zwischen dem Feuchtigkeitswert und einem eingestellten Feuchtigkeitsschwellenwert zu berechnen, das Kompensationswert-Bestimmungsmodul ausgebildet ist, um den Formaldehydkonzentrations-Kompensationswert des Formaldehydsensors (43) gemäß der vom Differenzberechnungsmodul berechneten zweiten Differenz zu bestimmen;
wenn die Vorrichtung den Temperatursensor (44) und den Feuchtigkeitssensor (45) umfasst, das Differenzberechnungsmodul ausgebildet ist, um die erste Differenz zwischen dem Temperaturwert und dem eingestellten Temperaturschwellenwert und die zweite Differenz zwischen dem Feuchtigkeitswert und dem eingestellten Feuchtigkeitsschwellenwert zu berechnen, das Kompensationswert-Bestimmungsmodul ausgebildet ist, um den Formaldehydkonzentrations-Kompensationswert des Formaldehydsensors (43) gemäß der ersten Differenz und der zweiten Differenz, die durch das Differenzberechnungsmodul berechnet wurden, zu bestimmen.

6. Vorrichtung nach Anspruch 5, wobei das Kompensationswert-Bestimmungsmodul Folgendes umfasst:
eine erste Kompensationsbestimmungseinheit oder eine zweite Kompensationsbestimmungseinheit oder die erste Kompensationsbestimmungseinheit und die zweite Kompensationsbestimmungseinheit; und
eine Einheit zur Bestimmung des Formaldehydkonzentrations-Kompensationswertes;
wenn das Differenzberechnungsmodul ausgebildet ist, um die erste Differenz zwischen dem Temperaturwert und der eingestellten Temperaturschwelle zu berechnen, umfasst das Kompensationswert-Bestimmungsmodul die erste Kompensationsbestimmungseinheit;
wenn das Differenzberechnungsmodul ausgebildet ist, um die zweite Differenz zwischen dem Feuchtigkeitswert und dem eingestellten Feuchtigkeitsschwellenwert zu berechnen, umfasst das Kompensationswert-Bestimmungsmodul die zweite Kompensationsbestimmungseinheit;
wenn das Differenzberechnungsmodul ausgebildet ist, um die erste Differenz zwischen dem Temperaturwert und dem eingestellten Temperaturschwellenwert und die zweite Differenz zwischen dem Feuchtigkeitswert und dem eingestellten Feuchtigkeitsschwellenwert zu berechnen, umfasst das Kompensationswert-Bestimmungsmodul die erste Kompensationsbestimmungseinheit und die zweite Kompensationsbestimmungseinheit;
die erste Kompensationswert-Bestimmungseinheit ausgebildet ist, um einen ersten Formaldehydkonzentrations-Kompensationswert des Formaldehydsensors (43) zu bestimmen, der der ersten Differenz zwischen dem Temperaturwert und dem eingestellten Temperaturschwellenwert entspricht, gemäß einer voreingestellten entsprechenden Beziehung zwischen der ersten Differenz und dem Formaldehydkonzentrations-Kompensationswert des Formaldehydsensors (43);
die zweite Kompensationswert-Bestimmungseinheit ausgebildet ist, um einen zweiten Formaldehydkonzentrations-Kompensationswert des Formaldehydsensors (43) zu bestimmen, der der zweiten Differenz zwischen dem Feuchtigkeitswert und dem eingestellten Feuchtigkeitsschwellenwert entspricht, gemäß einer voreingestellten entsprechenden Beziehung zwischen der zweiten Differenz und dem Formaldehydkonzentrations-Kompensationswert des Formaldehydsensors (43);
wenn das Differenzberechnungsmodul ausgebildet ist, um die erste Differenz zwischen dem Temperaturwert und der eingestellten Temperaturschwelle zu berechnen, die Formaldehydkonzentrations-Kompensationswert-Bestimmungseinheit ausgebildet ist, um den ersten Formaldehydkonzentrations-Kompensationswert, der durch die erste Kompensationswert-Bestimmungseinheit bestimmt wurde, als den Formaldehydkonzentrations-Kompensationswert des Formaldehydsensors (43) einzustellen;
wenn das Differenzberechnungsmodul ausgebildet ist, um die zweite Differenz zwischen dem Feuchtigkeitswert und dem eingestellten Feuchtigkeitsschwellenwert zu berechnen, die Formaldehydkonzentrations-Kompensationswert-Bestimmungseinheit ausgebildet ist, um den zweiten Formaldehydkonzentrations-Kompensationswert, der durch die zweite Kompensationswert-Bestimmungseinheit bestimmt wurde, als den Formaldehydkonzentrations-Kompensationswert des Formaldehydsensors (43) einzustellen; und
wenn das Differenzberechnungsmodul ausgebildet ist, um die erste Differenz zwischen dem Temperaturwert und dem eingestellten Temperaturschwellenwert und die zweite Differenz zwischen dem Feuchtigkeitswert und dem eingestellten Feuchtigkeitsschwellenwert zu berechnen, die Formaldehydkonzentrations-Kompensationswert-Bestimmungseinheit ausgebildet ist, um eine Summe des ersten Formaldehydkonzentrations-Kompensationswertes, der durch die erste Kompensationswert-Bestimmungseinheit bestimmt wird, und des zweiten Formaldehydkonzentrations-Kompensationswertes, der durch die zweite Kompensationswert-Bestimmungseinheit bestimmt wird, als den Formaldehydkonzentrations-Kompensationswert des Formaldehydsensors (43) einzustellen.

7. Luftreiniger, der die Vorrichtung zur Messung der Formaldehydkonzentration nach einem der Ansprüche 4 bis 6 umfasst.

## Revendications

1. Procédé de mesure de la concentration en formaldéhyde, comprenant :
la détermination d'une valeur initiale de la concentration en formaldéhyde mesurée par un capteur de formaldéhyde (43) ;
l'acquisition d'informations de température d'un environnement entourant le capteur de formaldéhyde (43), et la détermination d'une valeur de compensation de la concentration en formaldéhyde du capteur de formaldéhyde (43) selon les informations de température ; ou l'acquisition d'informations d'humidité de l'environnement entourant le capteur de formaldéhyde (43), et la détermination de la valeur de compensation de la concentration en formaldéhyde du capteur de formaldéhyde (43) selon les informations d'humidité ; ou l'acquisition des informations de température et des informations d'humidité de l'environnement entourant le capteur de formaldéhyde (43), et la détermination de la valeur de compensation de la concentration en formaldéhyde du capteur de formaldéhyde (43) selon les informations de température et des informations d'humidité ; et
la détermination d'une valeur cible de la concentration en formaldéhyde du capteur de formaldéhyde (43) selon la valeur de compensation de la concentration en formaldéhyde et la valeur initiale de la concentration en formaldéhyde ;
**caractérisé en ce qu'**il comprend en outre :
l'acquisition d'informations de paramètres de fonctionnement du capteur de formaldéhyde (43) dans une période prédéfinie, dans lequel les informations de paramètres de fonctionnement comprennent : la période totale de fonctionnement du capteur de formaldéhyde (43) dans la période préétablie, ou le nombre total d'opérations du capteur de formaldéhyde (43) dans la période préétablie, ou la période totale de fonctionnement du capteur de formaldéhyde (43) dans la période préétablie et le nombre total d'opérations du capteur de formaldéhyde (43) dans la période préétablie ; et
l'envoi d'un signal de commande correspondant au capteur de formaldéhyde (43) afin d'ordonner au capteur de formaldéhyde (43) d'effectuer un étalonnage du point zéro lorsque les informations de paramètres de fonctionnement satisfont à une condition préétablie ; et
le réétalonnage du point zéro par le capteur de formaldéhyde en réponse au signal de commande correspondant ;
dans lequel l'envoi du signal de commande correspondant au capteur de formaldéhyde (43) afin d'ordonner au capteur de formaldéhyde (43) d'effectuer l'étalonnage du point zéro, lorsque les informations de paramètre de fonctionnement satisfont à la condition préétablie, comprend :
lorsque les informations de paramètres de fonctionnement du capteur de formaldéhyde (43) dans la période préétablie sont la période de fonctionnement totale du capteur de formaldéhyde (43) dans la période préétablie, la comparaison de la période de fonctionnement totale du capteur de formaldéhyde (43) dans la période préétablie avec un seuil de période de fonctionnement totale établi pour obtenir un premier résultat de comparaison ; la détermination que les informations de paramètres de fonctionnement satisfont à la condition préétablie lorsque le premier résultat de comparaison est que la période de fonctionnement totale du capteur de formaldéhyde (43) dans la période préétablie est supérieure au seuil de période de fonctionnement totale établi ;
lorsque les informations de paramètres de fonctionnement du capteur de formaldéhyde (43) dans la période préétablie sont le nombre total d'opérations du capteur de formaldéhyde (43) dans la période préétablie, la comparaison du nombre total d'opérations du capteur de formaldéhyde (43) dans la période préétablie avec un seuil de nombre total d'opérations établi pour obtenir un second résultat de comparaison ; la détermination que les informations de paramètres de fonctionnement satisfont à la condition préétablie lorsque le second résultat de comparaison est que le nombre total d'opérations du capteur de formaldéhyde (43) dans la période préétablie est supérieur au seuil de nombre total d'opérations établi ;
lorsque les informations de paramètres de fonctionnement du capteur de formaldéhyde (43) dans la période préétablie comprennent la période totale de fonctionnement du capteur de formaldéhyde (43) dans la période préétablie et le nombre total d'opérations du capteur de formaldéhyde (43) dans la période préétablie, la comparaison de la période totale de fonctionnement du capteur de formaldéhyde (43) dans la période préétablie avec le seuil de fonctionnement total établi pour obtenir le premier résultat de comparaison, et la comparaison du nombre total d'opérations du capteur de formaldéhyde (43) dans la période préétablie avec le seuil de nombre total d'opérations établi pour obtenir le second résultat de comparaison ; et la détermination que les informations de paramètres de fonctionnement satisfont à la condition préétablie lorsque le premier résultat de comparaison est que la période de fonctionnement totale du capteur de formaldéhyde (43) dans la période préétablie est supérieure au seuil de période de fonctionnement totale établi, et le second résultat de comparaison est que le nombre total d'opérations du capteur de formaldéhyde (43) dans la période préétablie est supérieur au seuil de nombre total d'opérations établi.

2. Procédé selon la revendication 1, dans lequel les informations de température sont une valeur de température, et les informations d'humidité sont une valeur d'humidité ;
la détermination de la valeur de compensation de la concentration en formaldéhyde du capteur de formaldéhyde (43) selon les informations de température comprend : le calcul d'une première différence entre la valeur de température et un seuil de température établi, et la détermination de la valeur de compensation de la concentration en formaldéhyde du capteur de formaldéhyde (43) selon la première différence ;
la détermination de la valeur de compensation de la concentration en formaldéhyde du capteur de formaldéhyde (43) selon les informations d'humidité comprend : le calcul d'une seconde différence entre la valeur d'humidité et un seuil d'humidité établi, et la détermination de la valeur de compensation de la concentration en formaldéhyde du capteur de formaldéhyde (43) selon la seconde différence ;
la détermination de la valeur de compensation de la concentration en formaldéhyde du capteur de formaldéhyde (43) selon les informations de température et des informations d'humidité comprend : le calcul de la première différence entre la valeur de température et le seuil de température établi et la seconde différence entre la valeur d'humidité et le seuil d'humidité établi ; et la détermination de la valeur de compensation de la concentration en formaldéhyde du capteur de formaldéhyde (43) selon la première différence et la seconde différence.

3. Procédé selon la revendication 2, dans lequel
la détermination de la valeur de compensation de la concentration en formaldéhyde du capteur de formaldéhyde (43) selon la première différence comprend : la détermination d'une première valeur de compensation de la concentration en formaldéhyde du capteur de formaldéhyde (43) correspondant à la première différence entre la valeur de température et le seuil de température établi, selon une relation correspondante préétablie entre la première différence et la valeur de compensation de la concentration en formaldéhyde du capteur de formaldéhyde (43) ; et l'établissement de la première valeur de compensation de la concentration en formaldéhyde en tant que valeur de compensation de la concentration en formaldéhyde du capteur de formaldéhyde (43) ;
la détermination de la valeur de compensation de la concentration en formaldéhyde du capteur de formaldéhyde (43) selon la seconde différence comprend :
la détermination d'une seconde valeur de compensation de la concentration en formaldéhyde du capteur de formaldéhyde (43) correspondant à la seconde différence entre la valeur d'humidité et le seuil d'humidité établi, selon une relation correspondante préétablie entre la seconde différence et la valeur de compensation de la concentration en formaldéhyde du capteur de formaldéhyde (43) ; et l'établissement de la seconde valeur de compensation de la concentration en formaldéhyde en tant que valeur de compensation de la concentration en formaldéhyde du capteur de formaldéhyde (43) ;
la détermination de la valeur de compensation de la concentration en formaldéhyde du capteur de formaldéhyde (43) selon la première différence et la seconde différence comprend : la détermination de la première valeur de compensation de la concentration en formaldéhyde du capteur de formaldéhyde (43) correspondant à la première différence entre la valeur de température et le seuil de température établi, selon la relation correspondante préétablie entre la première différence et la valeur de compensation de la concentration en formaldéhyde du capteur de formaldéhyde (43) ; la détermination de la seconde valeur de compensation de la concentration en formaldéhyde du capteur de formaldéhyde (43) correspondant à la seconde différence entre la valeur d'humidité et le seuil d'humidité établi, selon la relation correspondante préétablie entre la seconde différence et la valeur de compensation de la concentration en formaldéhyde du capteur de formaldéhyde (43) ; et l'établissement d'une somme de la première valeur de compensation de la concentration en formaldéhyde et de la seconde valeur de compensation de la concentration en formaldéhyde en tant que valeur de compensation de la concentration en formaldéhyde du capteur de formaldéhyde (43).

4. Appareil de mesure de la concentration en formaldéhyde, comprenant :
un processeur (41) ;
une interface de communication (42) ;
un capteur de formaldéhyde (43) connecté électriquement au processeur (41) par le biais de l'interface de communication ;
un capteur de température (44), ou un capteur d'humidité (45), ou le capteur de température (44) et le capteur d'humidité (45), connectés électriquement au processeur (41) par le biais de l'interface de communication (42),
dans lequel le capteur de formaldéhyde (43) est configuré pour mesurer une concentration en formaldéhyde afin d'obtenir une valeur initiale de la concentration en formaldéhyde, et pour envoyer la valeur initiale de la concentration en formaldéhyde au processeur (41) ;
le capteur de température (44) est configuré pour collecter des informations de température d'un environnement entourant le capteur de formaldéhyde (43), et pour envoyer les informations de température au processeur (41) ;
le capteur de température (44) est configuré pour collecter des informations d'humidité de l'environnement entourant le capteur de formaldéhyde (43), et pour envoyer les informations d'humidité au processeur (41) ;
lorsque l'appareil comprend le capteur de température (44), le processeur (41) est configuré pour déterminer une valeur de compensation de la concentration en formaldéhyde du capteur de formaldéhyde (43) selon les informations de température qui sont reçues ;
lorsque l'appareil comprend le capteur d'humidité (45), le processeur (41) est configuré pour déterminer la valeur de compensation de la concentration en formaldéhyde du capteur de formaldéhyde (43) selon les informations d'humidité qui sont reçues ;
lorsque l'appareil comprend le capteur de température (44) et le capteur d'humidité (45), le processeur (41) est configuré pour déterminer la valeur de compensation de la concentration en formaldéhyde du capteur de formaldéhyde (43) selon les informations de température et les informations d'humidité qui sont reçues ; et
le processeur (41) est configuré pour déterminer une valeur cible de la concentration en formaldéhyde du capteur de formaldéhyde (43) selon la valeur de compensation de la concentration en formaldéhyde et la valeur initiale de la concentration en formaldéhyde qui est reçue ;
**caractérisé en ce que** l'appareil de mesure de la concentration en formaldéhyde comprend en outre :
un module d'acquisition d'informations de paramètres configuré pour acquérir des informations de paramètres de fonctionnement du capteur de formaldéhyde (43) dans une période prédéfinie, dans lequel les informations de paramètres de fonctionnement comprennent : la période totale de fonctionnement du capteur de formaldéhyde (43) dans la période préétablie, ou le nombre total d'opérations du capteur de formaldéhyde (43) dans la période préétablie, ou la période totale de fonctionnement du capteur de formaldéhyde (43) dans la période préétablie et le nombre total d'opérations du capteur de formaldéhyde (43) dans la période préétablie ;
le processeur (41) est configuré pour envoyer un signal de commande correspondant au capteur de formaldéhyde (43) afin d'ordonner au capteur de formaldéhyde (43) de réaliser un étalonnage du point zéro lorsque les informations de paramètres de fonctionnement acquises par le module d'acquisition d'informations de paramètres satisfont à une condition préétablie ; et
le capteur de formaldéhyde (43) est configuré pour réaliser un étalonnage du point zéro en réponse au signal de commande correspondant ;
dans lequel le processeur (41) comprend en outre un premier module de comparaison, ou un second module de comparaison, ou le premier module de comparaison et le second module de comparaison ;
lorsque les informations de paramètres de fonctionnement comprennent la période totale de fonctionnement du capteur de formaldéhyde (43) dans la période préétablie, le processeur (41) comprend en outre le premier module de comparaison ; lorsque les informations de paramètres de fonctionnement comprennent le nombre total d'opérations du capteur de formaldéhyde (43) dans la période préétablie, le processeur (41) comprend en outre le second module de comparaison ; lorsque les informations de paramètres de fonctionnement comprennent la période totale de fonctionnement du capteur de formaldéhyde (43) dans la période préétablie et le nombre total d'opérations du capteur de formaldéhyde (43) dans la période préétablie, le processeur (41) comprend en outre le premier module de comparaison et le second module de comparaison ;
le premier module de comparaison est configuré pour comparer la période totale de fonctionnement du capteur de formaldéhyde (43) dans la période préétablie avec un seuil de période de fonctionnement totale établi afin d'obtenir un premier résultat de comparaison ;
le second module de comparaison est configuré pour comparer le nombre total d'opérations du capteur de formaldéhyde (43) dans la période préétablie avec un seuil de nombre total d'opérations établi afin d'obtenir un second résultat de comparaison ;
lorsque le processeur (41) comprend en outre le premier module de comparaison, et lorsque le premier résultat de comparaison obtenu par le premier module de comparaison est que la période totale de fonctionnement du capteur de formaldéhyde (43) dans la période préétablie est supérieure au seuil de période de fonctionnement totale établi, le processeur (41) détermine que les informations de paramètres de fonctionnement acquises par le module d'acquisition d'informations de paramètres satisfont à la condition préétablie ;
lorsque le processeur (41) comprend en outre le second module de comparaison, et lorsque le second résultat de comparaison obtenu par le second module de comparaison est que le nombre total d'opérations du capteur de formaldéhyde (43) dans la période préétablie est supérieur au seuil de nombre total d'opérations établi, le processeur (41) détermine que les informations de paramètres de fonctionnement acquises par le module d'acquisition d'informations de paramètres satisfont à la condition préétablie ;
lorsque le processeur (41) comprend en outre le premier module de comparaison et le second module de comparaison, et lorsque le premier résultat de comparaison obtenu par le premier module de comparaison est que la période totale de fonctionnement du capteur de formaldéhyde (43) dans la période préétablie est supérieure au seuil de période totale de fonctionnement établi, et le second résultat de comparaison obtenu par le second module de comparaison est que le nombre total d'opérations du capteur de formaldéhyde (43) dans la période préétablie est supérieur au seuil de nombre total d'opérations établi, le processeur (41) détermine que les informations de paramètres de fonctionnement acquises par le module d'acquisition d'informations de paramètres satisfont à la condition préétablie.

5. Appareil selon la revendication 4, dans lequel les informations de température sont une valeur de température, les informations d'humidité sont une valeur d'humidité, et le processeur (41) comprend un module de calcul de différence et un module de détermination de valeur de compensation ;
lorsque l'appareil comprend le capteur de température (44), le module de calcul de différence est configuré pour calculer une première différence entre la valeur de température et un seuil de température établi, le module de détermination de valeur de compensation est configuré pour déterminer la valeur de compensation de la concentration en formaldéhyde du capteur de formaldéhyde (43) selon la première différence calculée par le module de calcul de différence ;
lorsque l'appareil comprend le capteur d'humidité (45), le module de calcul de différence est configuré pour calculer une seconde différence entre la valeur d'humidité et un seuil d'humidité établi, le module de détermination de valeur de compensation est configuré pour déterminer la valeur de compensation de la concentration en formaldéhyde du capteur de formaldéhyde (43) selon la seconde différence calculée par le module de calcul de différence ;
lorsque l'appareil comprend le capteur de température (44) et le capteur d'humidité (45), le module de calcul de différence est configuré pour calculer la première différence entre la valeur de température et le seuil de température établi et la seconde différence entre la valeur d'humidité et le seuil d'humidité établi, le module de détermination de valeur de compensation est configuré pour déterminer la valeur de compensation de la concentration en formaldéhyde du capteur de formaldéhyde (43) selon la première différence et la seconde différence calculées par le module de calcul de différence.

6. Appareil selon la revendication 5, dans lequel le module de détermination de valeur de compensation comprend :
une première unité de détermination de compensation, ou une seconde unité de détermination de compensation, ou la première unité de détermination de compensation et la seconde unité de détermination de compensation ; et
une unité de détermination de la valeur de compensation de la concentration en formaldéhyde ;
lorsque le module de calcul de différence est configuré pour calculer la première différence entre la valeur de température et le seuil de température établi, le module de détermination de valeur de compensation comprend la première unité de détermination de compensation ;
lorsque le module de calcul de différence est configuré pour calculer la seconde différence entre la valeur d'humidité et le seuil d'humidité établi, le module de détermination de valeur de compensation comprend la seconde unité de détermination de compensation ;
lorsque le module de calcul de différence est configuré pour calculer la première différence entre la valeur de température et le seuil de température établi et la seconde différence entre la valeur d'humidité et le seuil d'humidité établi, le module de détermination de valeur de compensation comprend la première unité de détermination de compensation et la seconde unité de détermination de compensation ;
la première unité de détermination de valeur de compensation est configurée pour déterminer une première valeur de compensation de la concentration en formaldéhyde du capteur de formaldéhyde (43) correspondant à la première différence entre la valeur de température et le seuil de température établi, selon une relation correspondante préétablie entre la première différence et la valeur de compensation de la concentration en formaldéhyde du capteur de formaldéhyde (43) ;
la seconde unité de détermination de valeur de compensation est configurée pour déterminer une seconde valeur de compensation de la concentration en formaldéhyde du capteur de formaldéhyde (43) correspondant à la seconde différence entre la valeur d'humidité et le seuil d'humidité établi, selon une relation correspondante préétablie entre la seconde différence et la valeur de compensation de la concentration en formaldéhyde du capteur de formaldéhyde (43) ;
lorsque le module de calcul de différence est configuré pour calculer la première différence entre la valeur de température et le seuil de température établi, l'unité de détermination de valeur de compensation de la concentration en formaldéhyde est configurée pour établir la première valeur de compensation de la concentration en formaldéhyde déterminée par la première unité de détermination de valeur de compensation en tant que valeur de compensation de la concentration en formaldéhyde du capteur de formaldéhyde (43) ;
lorsque le module de calcul de différence est configuré pour calculer la seconde différence entre la valeur d'humidité et le seuil d'humidité établi, l'unité de détermination de valeur de compensation de la concentration en formaldéhyde est configurée pour établir la seconde valeur de compensation de la concentration en formaldéhyde déterminée par la seconde unité de détermination de valeur de compensation en tant que valeur de compensation de la concentration en formaldéhyde du capteur de formaldéhyde (43) ; et
lorsque le module de calcul de différence est configuré pour calculer la première différence entre la valeur de température et le seuil de température établi et la seconde différence entre la valeur d'humidité et le seuil d'humidité établi, l'unité de détermination de valeur de compensation de la concentration en formaldéhyde est configurée pour établir une somme de la première valeur de compensation de la concentration en formaldéhyde déterminée par la première unité de détermination de valeur de compensation et de la seconde valeur de compensation de la concentration en formaldéhyde déterminée par la seconde unité de détermination de valeur de compensation, en tant que valeur de compensation de la concentration en formaldéhyde du capteur de formaldéhyde (43).

7. Purificateur d'air, comprenant l'appareil de mesure de la concentration en formaldéhyde selon l'une quelconque des revendications 4 à 6.
